# EUROPÄISCHE PATENTANMELDUNG

(11) **EP 3 270 609 A1**
(43) Veröffentlichungstag der Anmeldung: **17.01.2018**
(21) Anmeldenummer: 17169115.7
(22) Anmeldetag: 02.05.2017
(51) Int. Cl.: H04R 25/00

(54) **VERFAHREN ZUR FUNKTIONS- UND/ODER SITZÜBERPRÜFUNG EINES HÖRGERÄTES**

(30) Priorität: 14.07.2016 DE 102016212879
(71) Anmelder: Sivantos Pte. Ltd., Singapore 139959 (SG)
(72) Erfinder: HÜTTINGER, Simon, 91056 Erlangen (DE); PISCHEL, Judith, 90419 Nürnberg (DE); FRÖHLICH, Matthias, 91056 Erlangen (DE); DICKEL, Thomas, 96155 Buttenheim (DE)
(74) Vertreter: FDST Patentanwälte

(57) **Zusammenfassung**

Die Erfindung betrifft ein Verfahren zur Funktions- und/oder Sitzüberprüfung eines Hörgerätes, wobei in Abhängigkeit eines nutzerspezifischen Audiogramms eine Anzahl von Tönen in ein in einem Ohrkanal sitzendes Hörgerät eingespielt wird, wobei eine nutzerspezifische Reaktion bezüglich der Hörwahrnehmung des oder jeden eingespielten Tones erfasst wird, und wobei anhand der nutzerspezifischen Reaktion auf die Funktion- und/oder auf den Sitz des Hörgerätes im Ohrkanal geschlossen wird.

## Beschreibung

Die Erfindung betrifft ein Verfahren zur Funktions- und/oder Sitzüberprüfung eines Hörgerätes.

Weltweit ist eine Vielzahl von Menschen von Hörverlust und Schwerhörigkeit betroffen. Die Verringerung der eigenen Hörleistung wirkt sich negativ auf die Kommunikation und den Kontakt zu anderen Menschen aus. Hörgeräte ermöglichen ihren Anwendern, die ein vermindertes Hörvermögen aufweisen, wieder eine weitestgehend normale akustische Wahrnehmung.

Eine Fehlfunktion oder ein falscher Sitz eines Hörgerätes ist häufig ursächlich für die Ablehnung eines Hörgerätes, da die erwartete Verbesserung des Hörvermögens nicht auftritt und resultierende Fehlfunktionen als unangenehm empfunden werden. Entsprechend sind eine Verifizierung der Hörgerätefunktionalität und des richtigen Sitzes eines Hörgerätes von enormer Bedeutung, um die gewünschte Verbesserung des Hörvermögens und insbesondere eine weitgehend normale Hörwahrnehmung sicherzustellen. Derartige Überprüfungen werden üblicherweise regelmäßig bei einem Hörgeräteakustiker durchgeführt, um auf die falsche Verwendung bzw. den falschen Sitz des Hörgerätes festzustellen und zu korrigieren. Allerdings ist eine Überprüfung des Sitzes oder einer eventuellen Fehlfunktion des Hörgerätes durch den Hörgeräteträger selbst wünschenswert, wenn er beispielsweise Unregelmäßigkeiten in seiner Hörwahrnehmung feststellt und die Fehlerursache gerne kennen bzw. beheben möchte.

Eine solche Überprüfung der ordnungsgemäßen Funktion des Hörgerätes ist jedoch nicht ohne weiteres möglich. Insbesondere älteren Menschen oder Kindern fallen eine fachgerechte Bedienung und eine Überprüfung von Hörgerätefunktionen nicht leicht. So kann es bei unsachgemäßer Änderung von Hörgeräteeinstellungen durch den Hörgeräteträger selbst oder auch durch Eltern, Verwandtschaft oder Pflegepersonal zu einer weiteren Verschlechterung des Hörvermögens kommen, auch wenn lediglich der Sitz des Hörgerätes nicht korrekt ist.

Aus der DE 10 2013 225 760 A1 ist ein Hörgerät mit Sizterkennung und eine entsprechendes Verfahren bekannt, welches erkennt ob ein Hörgerätesystem oder eine Hörgerätekomponente korrekt in einem Ohr sitzt. Dazu wird gemäß der DE 10 2013 225 760 A1 ein akustisches Testsignal von einer Ausgabeeinrichtung ausgegeben und von einer Aufnahmeeinrichtung wieder empfangen. Das wieder empfangene Testsignal wird mit fest voreingestellten Referenzwerten von Referenzkriterien verglichen um so ein Statussignal zu erhalten. Mittels des Statussignals kann dann festgestellt werden ob das Hörgerätesystem korrekt sitzt.

Aus der DE 10 2005 034 380 B3 sind ein Hörgerät mit automatischer Ermittlung seines Sitzes im Ohr und entsprechendes Verfahren bekannt. Um gemäß der DE 10 2005 034 380 B3 den korrekten Sitz des Hörgeräts oder der Hörgerätekomponente in einem Gehörgang zu ermitteln, wird ein akustisches Messsignal in den Gehörgang abgegeben. Das im Gehörgang durch das Hörgerät oder die Hörgerätekomponente beeinflusste Messsignal wird aufgenommen und mit einem Referenzsignal verglichen. Aus dem Vergleich wird eine Information über den Sitz des Hörgeräts ermittelt. Diese Information wird mit einem Statusbericht an eine externe Einheit übermittelt. Somit kann insbesondere bei der Kinderversorgung eine objektive Information über den Sitz des Hörgeräts erhalten werden.

Der Erfindung liegt als eine Aufgabe zugrunde, ein Verfahren anzugeben, welches eine einfache und schnelle Überprüfung der Funktion und/oder des Sitzes eines Hörgerätes ermöglicht.

Diese Aufgabe der Erfindung wird erfindungsgemäß gelöst durch ein Verfahren zur Funktions- und/oder Sitzüberprüfung eines Hörgerätes, wobei in Abhängigkeit eines nutzerspezifischen Audiogramms eine Anzahl von Tönen in ein in einem Ohrkanal sitzendes Hörgerät eingespielt wird, wobei eine nutzerspezifische Reaktion bezüglich der Hörwahrnehmung des oder jeden eingespielten Tones erfasst wird, und wobei anhand der nutzerspezifischen Reaktion auf die Funktion- und/oder auf den Sitz des Hörgerätes im Ohrkanal geschlossen wird.

In einem ersten Schritt geht die Erfindung von der Tatsache aus, dass bei einer verschlechterten Hörwahrnehmung der Grund hierfür seitens des Hörgeräteträgers nicht ohne weiteres ermittelt werden kann. Der Hörgeräteträger kann nicht beurteilen, ob bei verringerter oder verschlechterter Hörwahrnehmung lediglich ein falscher Sitz verantwortlich ist, ob das Hörgerät - beispielsweise durch defekte Bauteile oder eine leere Batterie - in seiner Funktion beeinträchtigt ist oder ob sich das eigene Hörvermögen verschlechtert hat, so dass eine Änderung von Hörgeräteeinstellungen vorgenommen werden sollte.

Eine Überprüfung muss entsprechend bei einem Hörgeräteakustiker vorgenommen werden, was mit einem erhöhten Aufwand verbunden ist, der gerade für ältere Menschen eine ungewollte zusätzliche Belastung darstellt. Entsprechend kann es vorkommen, dass eine schlechte Hörwahrnehmung seitens des Hörgeräteträgers vorerst hingenommen und der Alltag so bestritten wird.

Die Erfindung begegnet dieser Problematik nun durch ein Verfahren, bei welchem auf Basis der Reaktion eines Hörgeräteträgers Aussagen bezüglich der Funktion und/oder des Sitzes des jeweiligen Hörgerätes getroffen werden können. Hierzu wird in Abhängigkeit eines nutzerspezifischen Audiogramms eine Anzahl von Tönen in ein in einem Ohrkanal sitzendes Hörgerät eingespielt. Dann wird eine nutzerspezifische Reaktion bezüglich der Hörwahrnehmung des oder jeden eingespielten Tones erfasst. Insbesondere wird beispielsweise erfasst, ob der Nutzer den oder jeden eingespielten Ton gehört hat. Anhand dieser Ergebnisse, also anhand der nutzerspezifischen Reaktion wird dann auf die Funktion- und/oder auf den Sitz des Hörgerätes im Ohrkanal geschlossen.

Mittels eines solchen Verfahrens wird es einem Hörgeräteträger ermöglicht, selbstständig, oder mit Hilfe von Freunden, Verwandten oder Pflegepersonal, eine Überprüfung seines Hörgerätes durchzuführen. Ein Besuch bei einem Hörgeräteakustiker ist hierzu nicht notwendig.

Die Anzahl der Töne wird in Abhängigkeit des jeweiligen nutzerspezifischen Audiogramms eingespielt. Ein Audiogramm beschreibt das subjektive Hörvermögen für Töne, also die frequenzabhängige Hörempfindlichkeit eines Menschen. Das Audiogramm ist nutzerspezifisch und illustriert das Hörvermögen durch die Darstellung der Hörschwelle bei verschiedenen Frequenzen. Die Hörschwelle zeigt hierbei an, wie schwach ein Ton werden darf, bevor er für die betreffende Person nicht mehr hörbar ist, oder im umgekehrten Fall, ab welchem Lautstärkepegel ein Ton spezifischer Frequenz wahrgenommen wird.

Wird im Falle einer Überprüfung der oder jeder in das Hörgerät eingespielte Ton von einem Hörgeräteträger deutlich wahrgenommen, liegt keine Fehlfunktion des Hörgerätes vor. Auch der Sitz im Ohrkanal ist in diesem Fall korrekt. Insbesondere wird dazu der Ton entsprechender Frequenz mit einer Lautstärke eingespielt, die oberhalb der durch das beispielsweise im Hörgerät abgespeicherte Audiogramm gegebenen spezifischen Hörschwelle liegt.

Insbesondere wird anhand der nutzerspezifischen Reaktion auf eine fehlerhafte Funktion und/oder auf einen fehlerhaften Sitz des Hörgerätes im Ohrkanal geschlossen. Eine fehlerhafte Funktion und/oder ein fehlerhafter Sitz des Hörgerätes sind - unter der Annahme, dass sich das Hörvermögen des Hörgeräteträgers nicht grundsätzlich verschlechtert hat - dann naheliegend, wenn der oder die Töne, die aufgrund des vorliegenden persönlichen Audiogramms eigentlich gehört werden müssten, nur leise oder auch gar nicht wahrgenommen werden. Der Hörgeräteträger erhält dann eine Benachrichtigung darüber, ob lediglich der Sitz des Hörgerätes geändert werden muss, oder ob die Funktion des Hörgerätes derart beeinträchtigt ist, dass eine Überprüfung bei einem Akustiker und gegebenenfalls eine Reparatur des Hörgerätes notwendig wird.

Besonders bevorzugt wird die nutzerspezifische Reaktion bezüglich der Hörwahrnehmung mittels eines Computers, insbesondere mittels eines mobilen Kommunikationsmittels erfasst. Unter einem mobilen Kommunikationsmittel werden hierbei all solche Kommunikationsmittel verstanden, mittels derer eine mobile, insbesondere drahtlose Sprach- oder Datenkommunikation möglich ist. Als mobile Kommunikationsmittel zur Funktions- und/oder Sitzüberprüfung eines Hörgerätes sind beispielsweise Laptops, Tablets und Smartphones geeignet.

Die Verwendung eines Smartphones ist hierbei insbesondere bevorzugt. Dazu wird ein Smartphone beispielsweise mit einem entsprechenden Programm, also einer App verwendet, welches in der Lage ist, die nutzerspezifische Reaktion zu erfassen und anschließend auszuwerten. Die Reaktion des Nutzers wird vorzugsweise durch eine Erkennung einer Spracheingabe erfasst. Alternativ bevorzugt erfolgt als Reaktion eine manuelle Eingabe des Hörgeräteträgers oder einer den Hörgeräteträger unterstützenden Zweitperson, die erkannt und zugeordnet bzw. ausgewertet wird.

Besonders vorteilhaft ist es, wenn die nutzerspezifische Reaktion bezüglich der Hörwahrnehmung automatisch erfasst wird. Eine solche automatische Erfassung erfolgt bevorzugt über einen Spracherkennungsalgorithmus, der die gesprochene Sprache automatisch erkennt und diese dann vorzugsweise ebenfalls automatisch einer Datenverarbeitung zugänglich macht. Die Sprachaufnahme erfolgt zweckmäßigerweise über ein Mikrofon an dem jeweiligen zur Erfassung der nutzerspezifischen Reaktion eingesetzten Kommunikationsgeräts.

Anhand der nutzerspezifischen Reaktion wird - wiederum vorzugsweise automatisch - auf die Funktion- und/oder auf den Sitz des Hörgerätes im Ohrkanal geschlossen. Es wird also automatisch ermittelt, ob das Hörgerät einwandfrei funktioniert und/oder korrekt im Ohrkanal sitzt, oder ob etwaige Änderungen des Sitzes und/oder der Hörgeräteinstellungen vorgenommen werden müssen. Die Ausgabe der anhand der nutzerspezifischen Reaktion ermittelten Ergebnisse erfolgt bevorzugt ebenfalls automatisch. Dem Hörgeräteträger wird hierbei automatisch übermittelt, ob und wenn ja welches Problem vorliegt, und wie dieses behoben werden kann.

Die Ergebnisse werden besonders bevorzugt über das Kommunikationsmittel ausgebeben. So kann beispielsweise auf dem Display eines Kommunikationsmittels wie beispielsweise einem Smartphone eine die Ergebnisse der Überprüfung visualisierende Benachrichtigung erscheinen, die dem Hörgeräteträger mitteilt, ob er das "Hörgerät neu positionieren" muss, ob eine "Verschmutzung" vorliegt, oder ob es sich um eine die Funktion beeinträchtigende "Beschädigung von Hörgerätebauteilen" handelt. Alternativ bevorzugt zu einer visualisierten Ausgabe erfolgt die Ausgabe als Sprachausgabe. Insbesondere erfolgt die Ausgabe über ein spezielles Signal, das im Hörgerät abgespielt wird.

Besonders bevorzugt werden die Ergebnisse der Hörgeräteüberprüfung automatisch an einen Hörgeräteakustiker übermittelt. Dieser kann die Ergebnisse in der entsprechenden Patientendatei ablegen und bei der nächsten Anpassungssitzung gegebenenfalls zu Optimierungs- und/oder Beratungszwecken nutzen.

Zur Überprüfung der Funktion und/oder des Sitzes können einzelne Töne und/ oder Tonsequenzen in das Hörgerät eingespielt werden. Alle Töne sind hierbei auf das nutzerspezifische Audiogramm abgestimmt und berücksichtigen die nutzerspezifische Hörschwelle sowohl hinsichtlich der Lautstäke des oder jeden Tons als auch hinsichtlich der Frequenz.

Bevorzugt wird zur Überprüfung der Funktion und/oder des Sitzes eine Tonsequenz mit Tönen verschiedener Frequenzen in das Hörgerät eingespielt. Die Tonfrequenz ist die Anzahl der Schwingungen einer Schallwelle pro Sekunde und beschreibt die Tonhöhe. Die Tonhöhe eines Tones ist hierbei nicht durch eine einzige feste Frequenz gegeben, sondern wird psychoakustisch wahrgenommen und das Ergebnis eines Zusammenspiels verschiedener unterschiedlicher Frequenzen sein. Weiter bevorzugt werden Töne verschiedener Frequenzen mit unterschiedlicher Lautstärke in das Hörgerät eingespielt werden. Die Lautstärke der eingespielten Töne wird hierbei vorzugsweise frequenzabhängig an eine nutzerspezifische individuelle Hörschwelle angepasst.

Zweckmäßigerweise wird der oder jeder Ton in einer Lautstärke unterhalb einer nutzerspezifischen Unbehaglichkeitsschwelle eingespielt. Die Unbehaglichkeitsschwelle bezeichnet denjenigen Schalldruck eines akustischen Signals, ab dem das Hören als unangenehm laut empfunden wird. Die Unbehaglichkeitsschwelle ist zweckmäßigerweise Teil des nutzerspezifischen Audiogramms und wird somit bei der Funktions- und/oder Sitzüberprüfung entsprechend berücksichtigt. Die Pegelwerte hängen jeweils von der Lautstärke und der Frequenz eine wahrgenommen Tons ab.

Zweckmäßigerweise wird der oder jeder Ton in einer Lautstärke oberhalb der nutzerspezifischen individuellen Hörschwelle eingespielt wird. So wird sichergestellt, dass der oder jeder Ton durch den Hörgeräteträger grundsätzlich hörbar wäre. Besonders bevorzugt wird also eine Anzahl von Tönen zwischen der persönlichen Hörschwelle und der Unbehaglichkeitsschwelle - dem Bereich der angenehmsten Lautheit - eingespielt. Der oder jeder Ton wird vorzugsweise in einer Lautstärke in einem Bereich zwischen10 dB und 50 dB oberhalb der nutzerspezifischen individuellen Hörschwelle eingespielt.

Weiter bevorzugt wird ein Ton einer festen Lautstärke in seiner Frequenz variiert. So kann erfasst werden, ab welcher Lautstärke ein Ton einer bestimmten Frequenz erfasst wird. Das Einspielen eines in der Frequenz variierenden Tones im Sinne eines "Wobbeltones" ist für Hörgeräteträger, die an einem sogenannten Hörsturz oder Tinnitus leiden, von Vorteil, da ein solcher Ton nicht als unangenehm empfunden wird.

Die Frequenz des oder jeden Tones liegt zweckmäßigerweise in einem Bereich zwischen 500 Hz und 1500 Hz. Die Frequenz des oder jeden Tones wird hierbei insbesondere dem Grad des Hörverlustes des Hörgeräteträgers angepasst. Zusätzlich wird das Alter des Hörgeräteträgers berücksichtigt, da sich die Hörschwelle mit zunehmendem Alter zu hohen Frequenzen verschiebt.

Weiter bevorzugt wird zur Überprüfung der Funktion und/oder des Sitzes des Hörgerätes ein Ton einer festen Frequenz in seiner Lautstärke variiert. Hierbei kann ermittelt werden, welche Frequenzen der Hörgeräteträger bei einer bestimmten Lautstärke wahrnimmt.

Nachdem der Hörgeräteträger die Ergebnisse der Überprüfung seines Hörgerätes erhalten hat, steht es ihm frei, möglicherweise notwendige Änderungen hinsichtlich der Positionierung des Hörgerätes vorzunehmen oder dieses zu säubern. Gegebenenfalls kann eine Änderung von Einstellungsparametern notwendig sein. Alternativ kann der Hörgeräteträger einen Termin bei einem Hörgeräteakustiker vereinbaren.

Nach erfolgter Änderung des Sitzes innerhalb des Ohrkanals und/oder nach einer Reinigung oder Reparatur des Hörgerätes kann das Verfahren erneut durchgeführt werden, um so sicherzustellen, dass das Hörgerät nun optimal sitzt und keine Fehlfunktionen mehr zu verzeichnen sind.

Im Folgenden wird ein Ausführungsbeispiel der Erfindung anhand einer Zeichnung näher erläutert.

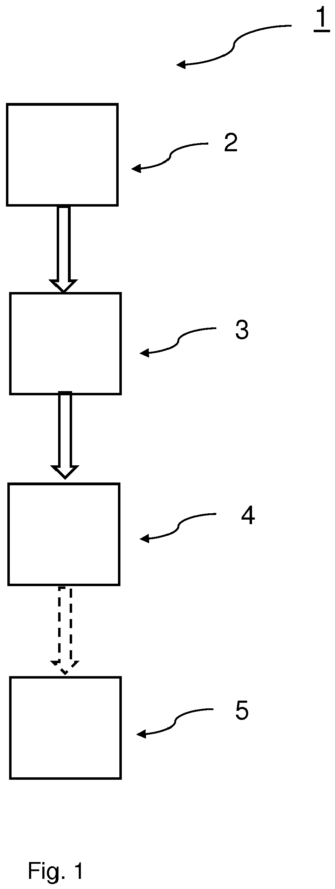

Fig. 1 zeigt den schematischen Verfahrensablauf 1 bei der Durchführung einer Funktions- und/oder Sitzüberprüfung eines Hörgerätes. Zuerst wird in Abhängigkeit eines nutzerspezifischen Audiogramms eine Tonsequenz von fünf Tönen in ein in einem Ohrkanal eines Hörgeräteträgers eingesetztes Hörgerät eingespielt. Die eingespielten Töne werden hierbei unterhalb der Unbehaglichkeitsschwelle und oberhalb der nutzerspezifischen Hörschwelle eingespielt. Die Töne haben vorliegend eine feste Lautstarke von 30 dB oberhalb der nutzerspezifischen individuellen Hörschwelle und werden in einem Frequenzbereich von 700 Hz bis 900 Hz in Schritten von 50 Hz nacheinander einzeln eingespielt (Schritt 2).

Dann benennt der Hörgeräteträger die Töne, die er hören konnte. Die Erfassung seiner nutzerspezifischen Wahrnehmung jedes einzelnen Tons erfolgt mittels eines Spracherkennungsprogramms eines Smartphones, welches die Reaktion automatisch aufzeichnet (Schritt 3).

Anhand dieser nutzerspezifischen Daten wird dann ermittelt, ob eine Fehlfunktion des Hörgerätes vorliegt und ob das Hörgerät richtig im Ohrkanal sitzt. Beispielhaft ist im Folgenden ausgeführt, welche Rückschlüsse anhand der wahrgenommenen Töne, also anhand der nutzerspezifischen Reaktion gezogen werden können (Schritt 4).

Hört der Hörgeräteträger alle fünf eingespielten Töne, so ist das Hörgerät richtig eingesetzt und funktioniert einwandfrei. Werden nur drei der vier Töne gehört, wird auf einen fehlerhaften Sitz geschlossen. Sind lediglich ein oder zwei Töne wahrnehmbar, so kann auf eine Verschmutzung von Bauteilen des Hörgerätes geschlossen werden. Ist es dem Hörgeräteträger nicht möglich, überhaupt einen Ton wahrzunehmen, so kann auf defekte Bauteile des Hörgerätes und/oder auf eine leere Batterie geschlossen werden. Dabei wird davon ausgegangen, dass das herangezogen Audiogramm aktuell ist, und sich das Hörvermögen des Hörgeräteträgers diesbezüglich nicht wesentlich verschlechtert hat.

Das jeweilige Ergebnis wird dem Hörgeräteträger dann automatisch ausgegeben, was vorliegend über das Display des eingesetzten Smartphones erfolgt. Anhand dieses Ergebnisses kann nun entschieden werden, ob lediglich eine Änderung der Position des Hörgerätes im Ohrkanal oder doch ein Besuch bei einem Akustiker nötig ist (Schritt 5).

Alternativ zu einer Tonsequenz kann selbstverständlich auch ein Ton einer festen Frequenz mit veränderlicher Laustärke eingespielt werden. Die Laustärke wird hierbei dann so lange erhöht, bis der Hörgeräteträger dessen Wahrnehmung bestätigt.

### Bezugszeichenliste

- 1: Verfahrensablauf
- 2: erster Schritt
- 3: zweiter Schritt
- 4: dritter Schritt
- 5: vierter Schritt

## Patentansprüche

1. Verfahren zur Funktions- und/oder Sitzüberprüfung eines Hörgerätes, wobei in Abhängigkeit eines nutzerspezifischen Audiogramms eine Anzahl von Tönen in ein in einem Ohrkanal sitzendes Hörgerät eingespielt wird, wobei eine nutzerspezifische Reaktion bezüglich der Hörwahrnehmung des oder jeden eingespielten Tones erfasst wird, und wobei anhand der nutzerspezifischen Reaktion auf die Funktion- und/oder auf den Sitz des Hörgerätes im Ohrkanal geschlossen wird.

2. Verfahren nach Anspruch 1, wobei anhand der nutzerspezifischen Reaktion auf eine fehlerhafte Funktion und/oder auf einen fehlerhaften Sitz des Hörgerätes im Ohrkanal geschlossen wird.

3. Verfahren nach Anspruch 1 oder 2, wobei die nutzerspezifische Reaktion bezüglich der Hörwahrnehmung mittels eines Computers, insbesondere mittels eins mobilen Kommunikationsmittels, erfasst wird.

4. Verfahren nach einem der vorhergehenden Ansprüche, wobei die nutzerspezifische Reaktion bezüglich der Hörwahrnehmung automatisch erfasst wird.

5. Verfahren nach einem der vorhergehenden Ansprüche, wobei anhand der nutzerspezifischen Reaktion automatisch auf die Funktion- und/oder den Sitz des Hörgerätes im Ohrkanal geschlossen wird.

6. Verfahren nach einem der vorhergehenden Ansprüche, wobei eine Tonsequenz mit Tönen verschiedener Frequenzen in das Hörgerät eingespielt wird.

7. Verfahren nach einem der vorhergehenden Ansprüche, wobei Töne verschiedener Frequenzen mit unterschiedlicher Lautstärke in das Hörgerät eingespielt werden.

8. Verfahren nach einem der vorhergehenden Ansprüche, wobei die Lautstärke der eingespielten Töne frequenzabhängig an eine nutzerspezifische individuelle Hörschwelle angepasst wird.

9. Verfahren nach einem der vorhergehenden Ansprüche, wobei der oder jeder Ton in einer Lautstärke unterhalb einer nutzerspezifischen Unbehaglichkeitsschwelle eingespielt wird.

10. Verfahren nach einem der vorhergehenden Ansprüche, wobei der oder jeder Ton in einer Lautstärke oberhalb der nutzerspezifischen individuellen Hörschwelle eingespielt wird.

11. Verfahren nach einem der vorhergehenden Ansprüche, wobei der oder jeder Ton in einer Lautstärke in einem Bereich zwischen10 dB und 50 dB oberhalb der nutzerspezifischen individuellen Hörschwelle eingespielt wird.

12. Verfahren nach einem der vorhergehenden Ansprüche, wobei ein Ton einer festen Lautstärke in seiner Frequenz variiert wird.

13. Verfahren nach einem der vorhergehenden Ansprüche, wobei die Frequenz des oder jeden Tones in einem Bereich zwischen 500 Hz und 1500 Hz liegen

14. Verfahren nach einem der vorhergehenden Ansprüche, wobei ein Ton einer festen Frequenz in seiner Lautstärke variiert wird.
